# EUROPEAN PATENT APPLICATION

(11) **EP 4 327 777 A1**
(43) Date of publication of application: **28.02.2024**
(21) Application number: 23174876.5
(22) Date of filing: 23.05.2023
(51) Int. Cl.: A61F 2/07, A61F 2/89

(54) **BRANCHED STENT GRAFT HAVING SUPPORT STENT**

(30) Priority: 25.05.2022 US 202263345585 P; 16.05.2023 US 202318197830
(71) Applicant: Medtronic Vascular Inc., Santa Rosa, CA 95403 (US)
(72) Inventor: GUSTAFSON, Sydney M., Santa Rosa, 95403 (US); MALSBARY, Todd S., Santa Rosa, 95403 (US); GALLIGAN, Darren T., Plymoth, o1752 (US); DAVISON, Justin P., Santa Rosa, 95403 (US); MYERS, Craig, Holister, 95023 (US)
(74) Representative: Zimmermann & Partner Patentanwälte mbB

(57) **Abstract**

A branched stent graft includes a main body aligning with a blood vessel in a radially expanded state and extending between proximal and distal ends. The branched stent graft includes a coupling extending from the main body proximate a branching blood vessel branching from the blood vessel in the radially expanded state. The main body includes a proximal body stent at least partially located between the proximal end of the main body and the coupling and a distal body stent at least partially located distal the coupling. The main body includes a surrounding region located between the proximal and distal body stents and extending along a main body circumference. The main body further includes a support stent located in the surrounding region and configured to maintain the main body in an open state when aligned with the blood vessel. The support stent imparts less radial force than the proximal and distal body stents.

## Description

### TECHNICAL FIELD

The present disclosure relates to a branched stent graft having a support stent.

### BACKGROUND

Endovascular procedures are a minimally invasive technique to deliver clinical treatments in a patient's vasculature. One example of a clinical treatment used in an endovascular procedure is deployment of a stent graft. A stent graft is an implantable device made of a tube-shaped surgical graft material and an expanding (e.g., self-expanding) stent frame. The stent graft is placed inside a patient's vasculature (e.g., blood vessel) to bridge a diseased blood vessel segment (e.g., an aneurismal, dissected, or torn blood vessel segment), and thereby excluding hemodynamic pressures of blood flow from the diseased blood vessel segment.

The diseased blood vessel segment may extend into vasculature having blood vessel bifurcations or segments of the aorta from which smaller branch arteries extend. For example, thoracic aortic aneurysms may include aneurysms present in the ascending thoracic aorta, the aortic arch, and/or branch arteries that emanate therefrom (e.g., the left subclavian, left common carotid, or the brachiocephalic arteries). In some cases, a branched stent graft can be used to treat such aneurysms. For example, a branched stent graft can be deployed in the main vessel (e.g., aortic arch) with a coupling extending therefrom and toward or into the branched artery (e.g., left subclavian), and a secondary stent graft can be deployed in the branched artery and connected to the coupling.

### SUMMARY

In an embodiment, a branched stent graft is disclosed. The branched stent graft includes a main body aligning with a blood vessel in a radially expanded state and extending between proximal and distal ends. The branched stent graft also includes a coupling extending from the main body proximate a branching blood vessel branching from the blood vessel in the radially expanded state. The main body includes a proximal body stent at least partially located between the proximal end of the main body and the coupling and a distal body stent at least partially located distal the coupling. The main body includes a surrounding region located between the proximal and distal body stents and extending along a main body circumference. The main body further includes a support stent located in the surrounding region and configured to maintain the main body in an open state when aligned with the blood vessel. The support stent imparts less radial force than the proximal and distal body stents.

In another embodiment, a branched stent graft is disclosed. The branched stent graft aligns with a blood vessel in a radially expanded state and extends between proximal and distal ends. The branched stent graft includes a coupling extending from the main body proximate a branching blood vessel branching from the blood vessel in the radially expanded state. The main body includes a proximal body stent at least partially located between the proximal end of the main body and the coupling and a distal body stent at least partially located distal the coupling. The proximal body stent has a proximal body stent diameter. The distal body stent has a distal body stent diameter. The main body includes a surrounding region located between the proximal and distal body stents and extending along a main body circumference. The main body further includes a support stent located in the surrounding region and configured to maintain the main body in an open state when aligned with the blood vessel. The support stent has a support stent diameter. The support stent diameter is less than the proximal body stent diameter and/or the distal body stent diameter.

In yet another embodiment, a branched stent graft is disclosed. The branched stent graft includes a main body aligning with a blood vessel in a radially expanded state and extending between proximal and distal ends. The branched stent graft includes a coupling extending from the main body proximate a branching blood vessel branching from the blood vessel in the radially expanded state. The main body includes a bare body stent portion secured to the proximal end of the main body and a distal body stent portion at least partially located distal the coupling. The main body includes a surrounding region located between the bare stent portion and the distal body stent portion and extending along a main body circumference. The main body further includes a loop stent portion located in the surrounding region and extending less than the main body circumference. The loop stent portion is configured to maintain the main body in an open state when aligned with the blood vessel.

### BRIEF DESCRIPTION OF THE DRAWINGS

Figure 1 is a schematic view of a branched stent graft deployed within an aorta.
Figure 2 is a side view of a delivery system configured to deliver a branched stent graft to a target deployment position within an aorta and to deploy the branched stent graft at the target deployment position.
Figure 3 is a circumferential view representing 360 degrees of a proximal portion of a branched stent graft according to a first embodiment.
Figure 4 is a circumferential view representing 360 degrees of a proximal portion of a branched stent graft according to a second embodiment.
Figure 5 is a circumferential view representing 360 degrees of a proximal portion of a branched stent graft according to a third embodiment.
Figure 6 is a circumferential view representing 360 degrees of a proximal portion of a branched stent graft according to a fourth embodiment.
Figure 7A is a first side view of a proximal portion of a branched stent graft according to a fifth embodiment.
Figure 7B is a second side view of the proximal portion of the branched stent graft rotated 180 degrees from the first side view according to the fifth embodiment.
Figure 8A is a first side view of a proximal portion of a branched stent graft according to a sixth embodiment.
Figure 8B is a second side view of the proximal portion of the branched stent graft rotated 180 degrees from the first side view according to the sixth embodiment.
Figure 9 is a side view of a proximal portion of a branched stent graft according to another embodiment.
Figure 10 depicts a side view of a stent graft including first and second stent loops.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful.

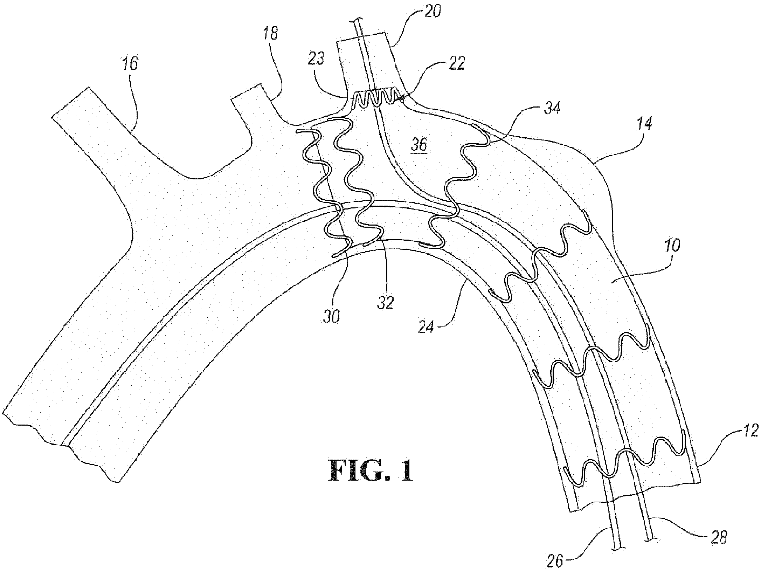

Figure 1 illustrates a schematic view of branched stent graft 10 deployed within aorta 12. Branched stent graft 10 may be used in an endovascular procedure to treat aneurysm 14 of the aorta 12. Aorta 12 branches into brachiocephalic artery 16, left common carotid artery 18, and left subclavian artery 20. As shown in Figure 1, branched stent graft 10 branches into left subclavian artery 20. In other embodiments, a branched stent graft may branch into other branches or two or more branches.

As shown in Figure 1, coupling 22 is external to main body 24 of branched stent graft 10 and is configured to branch into (e.g., align with and/or extend into) left subclavian artery 20 when branched stent graft 10 is deployed. In other embodiments, coupling 22 may be positioned on branched stent graft 10 to branch into other branches of the aorta 12, such as the brachiocephalic artery 16 or the left common carotid artery 18. Coupling 22 may be generally frustoconical-shaped with sloped side walls terminating at an opening. In other embodiments, the coupling 22 may be generally cylindrical. Annual stent 23 is coupled to the graft material of coupling 22 around the opening of coupling 22.

Prior to delivery of the branched stent graft 10, primary guidewire 26 may be inserted into aorta 12. Secondary guidewire 28 may also be inserted into aorta 12 separately from the primary guidewire 26, and into a target branch where coupling 22 is to be positioned (e.g., left subclavian artery 20 as shown on Figure 1). Primary guidewire 26 may track branched stent graft 10 along to a target deployment site, and secondary guidewire 28 may track a secondary stent graft (not shown) for deployment within left subclavian artery 20. Branched stent graft 10 and/or the secondary stent graft may be delivered with a stent graft delivery system.

During a surgical procedure, the stent graft delivery system may include primary and secondary lumens tracking along primary and secondary guidewires 26 and 28, respectively. Deployment of branched stent graft 10 may occur once branched stent graft 10 is situated at the target deployment site within aorta 14.

During deployment, main body 24 expands from a radially compressed state (not shown) outwardly to a radially expanded state (shown in Figure 1) and coupling 22 expands from a radially compressed state (not shown) outwardly to a radially expanded state (shown in Figure 1) with secondary guidewire 28 extending through coupling 22. Thereafter, a secondary stent graft (not shown) may track along secondary guidewire 28, through the coupling 22 and into left subclavian artery 20.

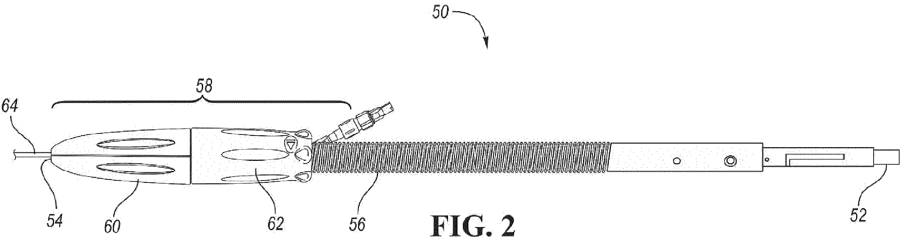

Figure 2 is a side view of delivery system 50 configured to deliver branched stent graft 10 to a target deployment position within aorta 12 and to deploy branched stent graft 10 at the target deployment position. Delivery system 50 extends between proximal end 52 and distal end 54. Threaded screw gear 56 extends between proximal end 52 and distal end 54 along the longitudinal axis of delivery system 50. Handle assembly 58 is configured to provide grip by a clinician using delivery system 50. As shown on Figure 2, handle assembly 58 includes front grip 60 and external slider 62. Front grip 60 may be fixed relative to screw gear 56, and external slider 62 may rotate about a threaded outer surface of screw gear 56 to move axially along the screw gear 56. For example, during deployment of branched stent graft 10, external slider 62 is rotated to move from distal end 54 toward proximal end 52. External slider 62 is operatively coupled to stent graft cover 64 (e.g., a sheath or a lumen) surrounding branched stent graft 10, thereby permitting the cover to retract with axial movement of external slider 62, thus permitting stent graft cover 10 to radially expand from the radially compressed state to the radially expanded state at the target deployment site within aorta 12.

As shown in Figure 1, branched stent graft 10 includes bare stent 30. Valley portions of bare stent 30 are secured to a proximal portion of a graft material of branched stent graft 10. Bare stent 30 may be stitched, sutured, or otherwise secured to a surface (e.g., inner surface or outer surface) of main body 24. The graft material may be formed from any blood-impermeable material, such as low-porosity woven or knit polyester, DACRON material, expanded polytetrafluoroethylene, polyurethane, and silicone. The graft material may also be formed from a natural material, such as pericardium or another membranous tissue such as intestinal submucosa. All bare stent 30 except for the depicted valley portions is unattached to the graft material. Bare stent 30 may be referred to as a free flow stent or a proximal stent. Bare stent 30 extends outside of main body 24 such that the extending portion may anchor to the inner walls of a blood vessel (e.g., aorta 12).

Branched stent graft 10 further includes a proximal body stent 32 located adjacent and distal to bare stent 30 and a distal body stent 34 located distal to proximal body stent 32. Each stent may be formed from a self-expanding or spring material, such as nickel-titanium alloy (Nitinol), stainless steel, a pseudo-elastic metal such as a nickel titanium alloy or nitinol, various polymers, or a so-called super alloy, which may have a base metal of nickel, cobalt, chromium, or other metal, or other suitable material. The stents described herein may be independent stent rings (e.g., formed in a single, closed loop) having a sinusoidal or zig-zag shape of alternating peaks and valleys with legs therebetween. The amplitude of the peaks and valleys may be constant or variable. As described in further detail below, portions of the stents may have non-sinusoidal portions, for example near or around coupling 22.

Coupling 22 is disposed between proximal body stent 32 and distal body stent 34. Surrounding region 36 extends axially between proximal body stent 32 and distal body stent 34 and radially around the graft material from a first side of coupling 22 to an opposing, second side of coupling 22.

Surrounding region 36 is configured to provide flexibility to the positioning of coupling 22 such that coupling 22 is configured to move and/or shift into a positioning location relative to a desired branch of aorta 12. For example, coupling 22 may have flexibility to accommodate a relatively high degree (e.g., 30 degrees) of off-positioning of branched stent graft 100 in either the proximal, distal, anterior, or posterior directions. This flexibility is beneficial to accommodate variations between different patients' anatomy in the branches of aorta 12. Such flexibility may also accommodate target placement of a secondary stent graft without compromising the positioning of coupling 22 during deployment or the location of coupling 22 along branched stent graft 10.

However, surrounding region 36 between proximal body stent 32 and distal body stent 34 and coupling 22 may cause an inability for branch stent graft 10 to conform around the aortic arch of aorta 12. This potential issue may be acute with patients having tortuous anatomies. This potential issue of lack of conformability may also cause patency concerns, for example, with respect to relatively small diameter branched stent grafts. The relatively small diameter may be any of the following diameters or in a range of any two of the following diameters: 6, 7, 8, 9, 10, 11, 12, 13, 14, 15, 16, 17, 18, 19, and 20 millimeters. Patency relates to maintaining the branched stent graft in an open condition (e.g., without an obstruction). In light of the foregoing, what may be beneficial is a support stent for branched stent grafts that encourages branched stent graft conformability (e.g., the branched stent graft conforms to native anatomy) and/or patency (e.g., at least 50% of the available area of the branched stent graft remains open) without sacrificing coupling location flexibility. One or more embodiments provide a branched stent graft including a support stent configured to maintain conformability and/or patency of the branched stent graft. In addition, the support stent does not sacrifice coupling location flexibility.

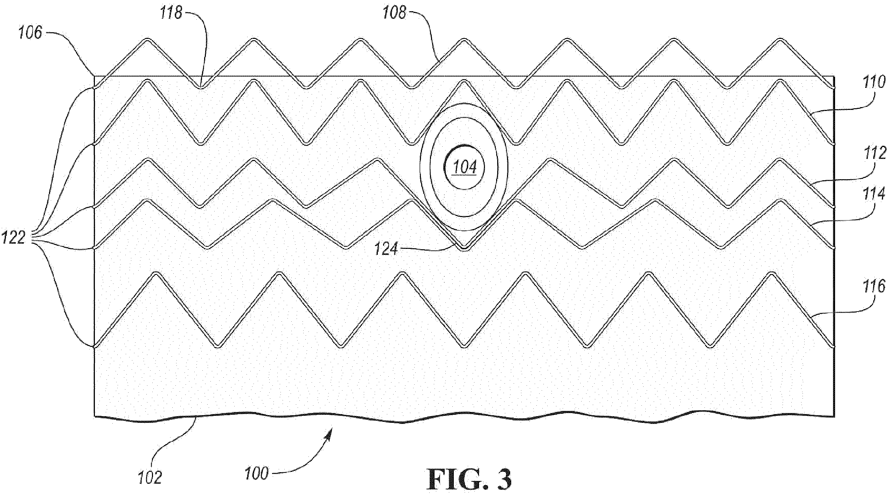

According to a first embodiment, Figure 3 is a circumferential view representing 360 degrees of proximal portion 100 of a branched stent graft unwrapped and laid flat. Proximal portion 100 includes main body 102 and coupling 104 extending from main body 102. Main body 102 aligns with a blood vessel (e.g., the aorta) in a radially expanded state and includes proximal end 106 and distal end (not shown). Coupling 104 extends from main body 102 aligning with a branching blood vessel (e.g., brachiocephalic artery, left common carotid artery, or left subclavian artery of the aorta) in the radially expanded state.

Main body 102 includes bare stent 108, proximal body stent 110, support stent 112, first distal body stent 114, and second distal body stent 116. Bare stent 108, proximal body stent 110, support stent 112, first distal body stent 114, and second distal body stent 116 may each be stitched, sutured, or otherwise secured to a surface (e.g., inner surface or outer surface) of main body 102. Bare stent 108 has valley portions 118 secured to proximal end 106 of main body 102. As shown in Figure 3, proximal body stent 110 is directly adjacent bare stent 108 with no intervening stents therebetween. First distal body stent 114 is located distal coupling 104. Second distal body stent 116 is located distal first distal body stent 114. Proximal body stent 110 may improve the seal of proximal end 106 with the vessel wall. Proximal body stent 110, first distal body stent 114, and second distal body stent 116 are configured to reinforce and give strength to main body 102. Main body 102 includes a surrounding region located between proximal body stent 110 and first distal body stent 114. The surrounding region extends along a main body circumference.

Main body 102 includes support stent 112 located in the surrounding region. Support stent 112 imparts less radial force than proximal and distal body stents 110 and 116. Support stent 112 is configured to maintain main body 102 in an open state when aligned with the primary blood vessel. Support stent 112 is configured to provide additional support for the branched stent graft in the surrounding region. As coupling 104 bends and flexes during placement with a secondary treated vessel, support stent 112 permits coupling 104 to maintain flexibility without sacrificing the structural integrity of main body 102. Support stent 112 permits relative flexing of coupling 104 relative to main body 102. Support stent 112 may also improve the seal of proximal end 106 with the vessel wall.

Support stent 112 may have a smaller diameter or thickness than proximal body stent 110, first distal body stent 114, and/or second distal body stent 116. The diameter of support stent 112 may be any of the following diameters or in a range of any two of the following diameters: 0.12, 0.15, 0.20, 0.25, 0.30, 0.35, and 0.38 millimeters. The diameter of proximal body stent 110, first distal body stent 114, and/or second distal body stent 116 may be any of the following diameters or in a range of any two of the following diameters: 0.010, 0.050, 0.10, 0.15, 0.20, 0.25, 0.30, 0.35, 0.40, 0.45, 0.50, 0.55, 0.60, and 0.635 millimeters. The diameter differential is configured to provide the proper balance of allowing flexibility while maintaining structural integrity. In one or more embodiments, the support stent has a smaller diameter than one or more of the body stents. The thinner diameter of the support stent may be configured to not interfere with the profile of the branched stent graft or significantly add radial force. Support stent 112 may be formed of a wire formed metal material. Alternatively, support stent 112 and body stent 114 may be formed of a laser cut metal material such that they are combined to form one continuous piece of metal.

As shown at the left and right edges in the circumferential view shown in Figure 3, valleys 122 of bare stent 108, proximal body stent 110, support stent 112, first distal body stent 114, and second distal body stent 116 are circumferentially aligned at a position opposite coupling 104 with corresponding peaks of other stents to provide conformability by nesting the corresponding peaks when main body 102 is bent in a tortuous anatomy. Support stent 112 includes valley 124 is aligned with coupling 104 to accommodate a cuff region surrounding coupling 104 to provide a region for flexing and deflection of coupling 104 without interference from support stent 112. Valley 124 contacts corresponding valley of first distal body stent 114. These valleys (or their adjacent legs) may be connected via a stent connector (e.g., a weld or a crimp tube). The corresponding peaks and valleys of support stent 112 and first distal body stent 114 adjacent the contacting valley may be out of phase to accommodate the longer sides of valley 124 to avoid coupling 104. As shown in Figure 3, support stent 112 extends along an entirety of the main body circumference.

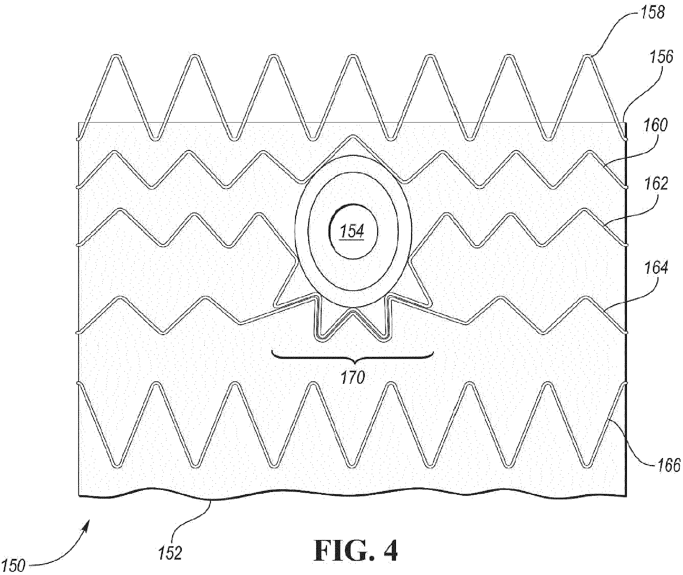

Figure 4 is a circumferential view representing 360 degrees of proximal portion 150 of a branched stent graft according to a second embodiment. Proximal portion 150 includes main body 152 and coupling 154 extending from main body 152. Main body 152 includes bare stent 158, proximal body stent 160, support stent 162, first distal body stent 164, and second distal body stent 166, which are connected to main body 152 as set forth herein and have the functionality as set forth herein. Main body 102 includes surrounding region located between proximal body stent 160 and first distal body stent 164. Surrounding region extends along a main body circumference.

Main body 152 includes support stent 162 located in the surrounding region. Support stent 152 may have a smaller diameter or thickness than proximal body stent 160, first distal body stent 164, and/or second distal body stent 166 as otherwise set forth herein. Support stent 162 and first distal body stent 164 share contact region 170. In contact region 170, support stent 162 and first distal body stent 164 has a star shape with several peaks. The star shaped formation may provide additional support to the cuff region without diminishing the flexibility of the cuff region. In contact region 170, support stent 162 and first distal body stent 164 may be connected via a stent connection (e.g., a weld or a crimp tube). The support stent 162 and the first distal body stent 164 may be connected proximate a distal end of the coupling 154. In another embodiment, the support stent 162 may be connected to the proximal body stent 160 in a similar manner, but proximate a proximal end of the coupling 154.

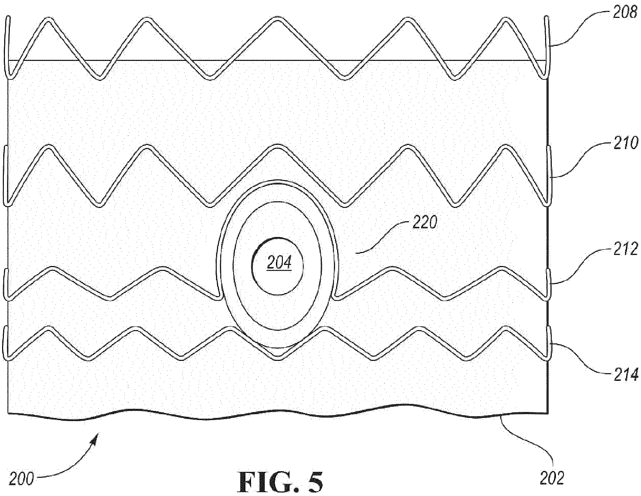

Figure 5 is a circumferential view representing 360 degrees of proximal portion 200 of a branched stent graft according to a third embodiment. Proximal portion 200 includes main body 202 and coupling 204 extending from main body 202. Main body 202 includes bare stent 208, proximal body stent 210, support stent 212, and distal body stent 214, which are connected to main body 202 as set forth herein and have the functionality as set forth herein. Main body 202 includes a surrounding region located between proximal body stent 210 and first distal body stent 214. The surrounding region extends along a main body circumference.

Main body 202 includes support stent 212 located in the surrounding region. Support stent 212 extends partially between coupling 204 and proximal body stent 210. Support stent 212 includes curved portion 220 having a profile conforming with the profile of coupling 204. The profile of curved portion 220 may provide additional support to the cuff region without diminishing the flexibility of the cuff region. Support stent 212 may have a smaller diameter or thickness than proximal body stent 210 and/or first distal body stent 214 as otherwise set forth herein. While the embodiment of Figure 5 shows the support stent 212 extending partially between coupling 204 and proximal body stent 210, in another embodiment it may extend in a similar manner between the coupling 204 and the first distal body stent 214.

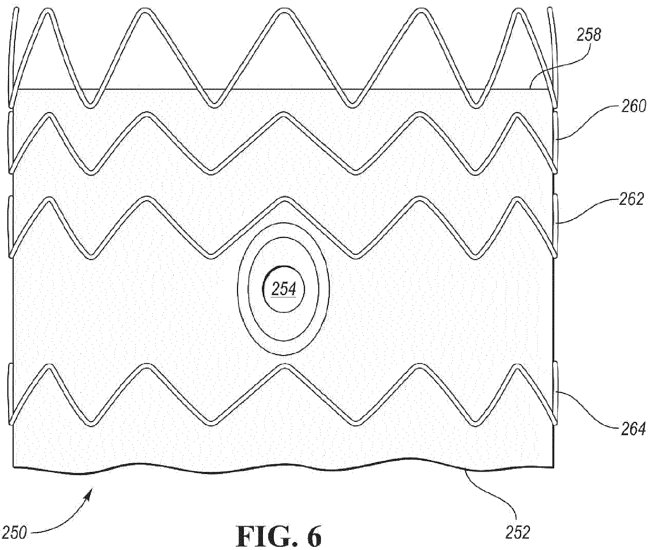

Figure 6 is a circumferential view representing 360 degrees of proximal portion 250 of a branched stent graft according to a fourth embodiment. Proximal portion 250 includes main body 252 and coupling 254 extending from main body 252. Main body 252 includes bare stent 258, proximal body stent 260, support stent 262, and distal body stent 264, which are connected to main body 252 as set forth herein and have the functionality as set forth herein. Main body 252 includes a surrounding region located between proximal body stent 260 and first distal body stent 264. The surrounding region extends along a main body circumference.

Main body 252 includes support stent 262 located in the surrounding region. Support stent 262 extends partially between coupling 264 and proximal body stent 260. Support stent 212 may have a smaller diameter or thickness than proximal body stent 210 and/or first distal body stent 214 as otherwise set forth herein. As shown in this embodiment, a majority of support stent 262 is proximal coupling 254. Support stent 262 may have a sinusoidal shape throughout its length and may have a constant amplitude between the peaks and valleys.

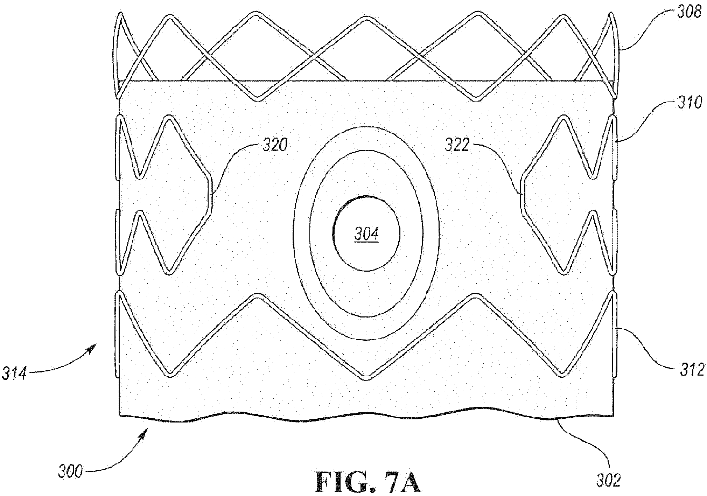

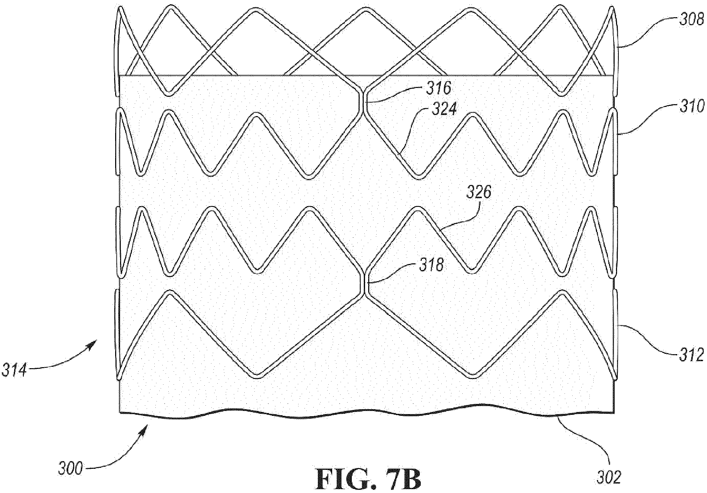

Figure 7A is a first side view of proximal portion 300 of a branched stent graft according to a fifth embodiment. Figure 7B is a second side view of proximal portion 300 of the branched stent graft rotated 180 degrees from the first side view according to the fifth embodiment. The first and second side views together represent an entire circumferential view of proximal portion 300. Proximal portion 300 includes main body 302 and coupling 304 extending from main body 302. Main body 302 aligns with a blood vessel (e.g., the aorta) in a radially expanded state and includes proximal end 306 and distal end (not shown). Coupling 304 extends from main body 302 aligning with a branching blood vessel (e.g., brachiocephalic artery, left common carotid artery, or left subclavian artery of the aorta) in the radially expanded state.

Main body 302 includes bare stent portion 308, loop stent portion 310, and distal body stent 312, which collectively form continuous stent 314 connected to main body 302 as set forth herein. Bare stent portion 308 is connected to loop stent portion 310 through proximal bridge portions 316, where a valley of the bare stent portion 308 is connected to a peak of the loop stent portion 310. Loop stent portion 310 and distal body stent 312 are connected to each other through distal bridge portions 318, where a valley of the loop stent portion 310 is connected to a peak of the distal body stent 312. In the embodiment shown, there is only a single bridge portion 316, 318 between the loop stent portion 310 and the bare stent portion 308 or distal body stent 312 and the connection is opposite the coupling 304 (e.g., 180 circumferentially offset). However, in other embodiments, there may be multiple proximal and/or distal bridge portions or connections between two portions. If there are multiple bridge portions, they may be evenly spaced about the circumference of the main body 302. The connections between the bare stent portion 308, the loop stent portion 310, and the distal body stent 312 may be via weld, crimp, adhesive, or any other suitable joining mechanism. In another embodiment, the three portions may be originally formed a single integral piece (e.g., by laser cutting).

Loop stent portion 310 includes first and second ends 320 and 322 and first and second sides 324 and 326. Loop stent portion 310 is configured to serve a dual function of a seal stent and a body stent. Loop stent portion 310 is also configured to terminate at first and second ends 320 and 322 short of a cuff region around coupling 304, thereby providing a region for flexing and deflection of coupling 304 without interference from loop stent portion 310. Loop stent portion 310 extends less than a circumference of main body 302. Loop stent portion 310 may have a smaller diameter or thickness than bare stent portion 308 and/or distal body stent portion 312 at such differential diameters as set forth herein.

The valleys of side 324 align with the peaks of side 326. The peaks of side 324 align with the valleys of side 326. These alignment features may repeat for the entire circumference between ends 320 and 322. As shown Figure 7A, sides 320 and 322 form flat shapes that vertically align with each other. Sides 324 and 326 and ends 320 and 322 form a closed loop configured to support coupling 304. The peaks of side 324 either align with a peak or a valley of bare stent portion 308 such that amplitude of side 324 is twice the amplitude of bare stent portion 308. The valleys of side 326 either align with a peak or a valley of distal body stent portion 312 such that the amplitude of side 326 is twice the amplitude of distal body stent portion 312. As shown in Figure 7A, the ends 320 and 322 are spaced apart from coupling 304 such that the ends 320 and 322 terminate before the peaks of distal body stent portion 312 on both sides of coupling 304. The peaks on side 324 adjacent to ends 320 and 322 align with peaks of bare stent portion 308. The valleys on side 326 adjacent to ends 320 and 322 align with valleys of distal body stent portion 312.

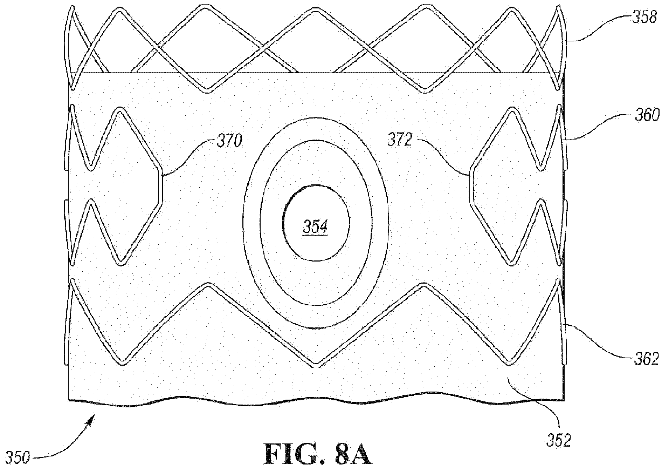

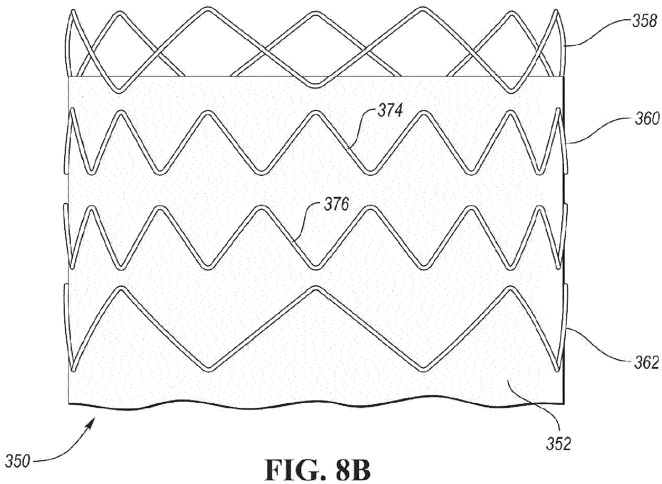

Figure 8A is a first side view of proximal portion 350 of a branched stent graft according to a sixth embodiment. Figure 8B is a second side view of proximal portion 350 of the branched stent graft rotated 180 degrees from the first side view according to the sixth embodiment. The first and second side views together represent an entire circumferential view of proximal portion 350.

Proximal portion 350 includes main body 352 and coupling 354 extending from main body 352. Main body 352 includes bare stent 358, loop stent 360, and distal body stent 362. As shown in Figures 8A and 8B, loop stent 360 is continuous, but in other embodiments, loop stent 360 may include one or more discontinuities. Loop stent 360 includes first and second ends 370 and 372 and first and second sides 374 and 376. Loop stent 360 is configured to serve a dual function of a seal stent and a body stent. Loop stent 360 is also configured to terminate at first and second ends 370 and 372 short of a cuff region around coupling 354, thereby providing a region for flexing and deflection of coupling 354 without interference from support stent 360. Loop stent 360 extends less than a circumference of main body 352. Loop stent 360 is spaced apart from bare stent 358 and distal body stent 362 (e.g., the three stents are only connected by the graft material). Loop stent 360 may have a smaller diameter or thickness than bare stent 358 and/or distal body stent 362 at such differential diameters as set forth herein.

The valleys of side 374 align with the peaks of side 376. The peaks of side 374 align with the valleys of side 376. These alignment features may repeat for the entire circumference between ends 370 and 372. As shown Figure 8A, sides 370 and 372 form flat shapes that vertically align with each other. Sides 374 and 376 and ends 370 and 372 form a closed loop configured to support coupling 354. The peaks of side 374 either align with a peak or a valley of bare stent 358 such that amplitude of side 374 is twice the amplitude of bare stent 358. The valleys of side 376 either align with a peak or a valley of distal body stent 362 such that the amplitude of side 376 is twice the amplitude of distal body stent 362. As shown in Figure 8A, the ends 370 and 372 are spaced apart from coupling 354 such that the ends 370 and 372 terminate before the peaks of distal body stent 362 on both sides of coupling 354. The peaks on side 374 adjacent to ends 370 and 372 align with peaks of bare stent portion 358. The valleys on side 376 adjacent to ends 370 and 372 align with valleys of distal body stent portion 362.

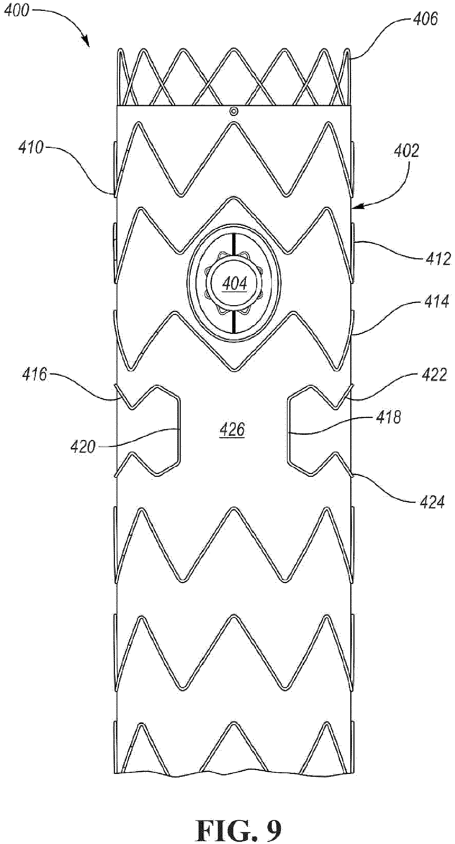

Figure 9 is a side view of proximal portion 400 of a branched stent graft according to another embodiment. Proximal portion 400 includes main body 402 and coupling 404 extending from main body 402. Main body 402 includes bare stent 406, seal stent 410, support stent 412, and distal body stent 414, which are connected to main body 402 as set forth herein and have the functionality as set forth herein. The stents at the proximal end may have any configuration as disclosed in previous embodiments (e.g., Figures 3 to 8B).

The branched stent graft of Figure 9 further includes stent loop 416 disposed distal body stent 414. Stent loop 416 includes first and second ends 418 and 420 and first and second sides 422 and 424, collectively forming a continuous loop. In other embodiments, stent loop 416 may be discontinuous (e.g., two or more segments that are each closed but separated by a gap in a circumferential direction). Stent loop 416 is configured to terminate at first and second ends 418 and 420 short of fenestration window 426. Stent loop 416 extends less than a circumference of main body 402. Stent loop 416 may have the same or a similar diameter or thickness as bare stent 406 and/or distal body stent 414. Stent loop 416 may be attached to distal body stem 414 and/or the stent immediately distal to stent loop 416 (e.g., a similar design as shown in Figure 7B but further distal the proximal end).

In one embodiment, the branched stent graft of Figure 9 is delivered with a stent graft delivery system to a target deployment site within an aorta. The branched stent graft is then deployed at the target deployment site within the aorta. Coupling 404 may be deployed such that it aligns with brachiocephalic artery; and fenestration window 426 (or two spaced apart fenestration windows) may align with left common carotid artery and/or left subclavian artery of the aorta such that fenestration window 426 (or two spaced apart fenestration windows) may be used to perfuse the left common carotid artery and/or left subclavian artery using in situ fenestration. In one or more embodiments, the fenestration window may be used with or without any branches (e.g., couplings). Two or more loop stents may be used to form two or more fenestration windows to enable multiple in situ fenestrations in one stent graft region. In this embodiments, two fenestration windows may be axially spaced to align with the left common carotid artery and the left subclavian artery, and circumferentially aligned or offset depending on the patient's anatomy. A branch graft may be coupled to an opening created by an in situ fenestration and extend into the left common carotid artery and/or the left subclavian artery to perfuse them.

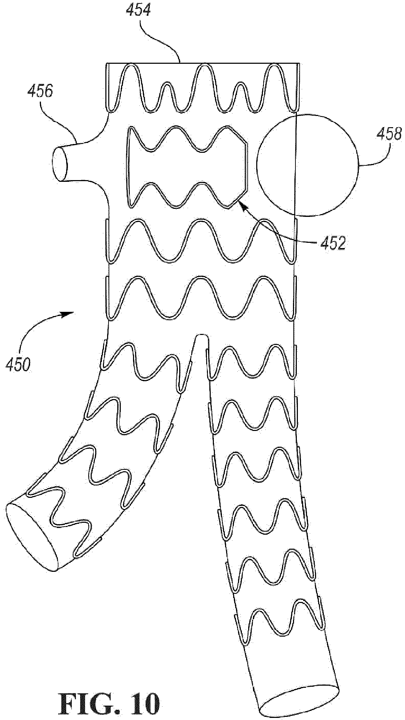

Figure 10 depicts a side view of stent graft 450 including first stent loop 452 and second stent loop (not shown) opposite first stent loop 452 on main body 454. The first and second stent loops may be aligned with each other on opposite sides of stent graft 450. In another embodiment, the first and second loops are offset each other on opposite sides of stent graft 450. Coupling 456 is disposed between a first gap between the first and second stent loops. Fenestration window 458 is disposed between a second gap between the first and second stent loops. Coupling 456 and fenestration window 458 may be aligned with each other on opposite sides of stent graft 450. Coupling 456 and fenestration window 458 may be offset each other on opposite sides of stent graft 450. During delivery, coupling 456 may be aligned on the one side with a pre-created branch within the patient's anatomy for renal access and fenestration window 458 may be aligned on the other opposing side for in situ fenestration for renal access. Fenestration window 458 may have a relatively large width to permit a signification portion of the population to obtain renal vascularization using stent graft 450. This embodiment may have the benefit of permitting vascularization to the renals for disease up to the height of the renal arteries. In one or more embodiments, stent graft 450 may include two couplings or two fenestration windows (e.g., with the same loop stents between them) instead of one coupling and one fenestration window.

While embodiments are described herein with respect to a branched stent graft, aspects of these embodiments may also be used in unbranched stent grafts (e.g., a cylindrical stent graft or a tubular stent graft). For example, any of the proximal body stent, the support stent, and/or the distal body stent may be incorporated into an unbranched stent graft as described herein.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

Aspects and embodiments of the invention may be defined by the following clauses.

Clause 1. A branched stent graft comprising:
a main body aligning with a blood vessel in a radially expanded state and extending between proximal and distal ends; and
a coupling extending from the main body proximate a branching blood vessel branching from the blood vessel in the radially expanded state;
the main body including a proximal body stent at least partially located between the proximal end of the main body and the coupling and a distal body stent at least partially located distal the coupling, the main body including a surrounding region located between the proximal and distal body stents and extending along a main body circumference, the main body further including a support stent located in the surrounding region and configured to maintain the main body in an open state when aligned with the blood vessel, and the support stent imparts less radial force than the proximal and distal body stents.

Clause 2. The branched stent graft of clause 1, wherein the support stent extends along an entirety of the main body circumference.

Clause 3. The branched stent graft of clause 1, wherein the support stent extends partially between the coupling and the proximal body stent or the distal body stent.

Clause 4. The branched stent graft of clause 1, wherein a contacting support stent portion of the support stent contacts a contacting body stent portion of the proximal body stent or the distal body stent in a contact region.

Clause 5. The branched stent graft of clause 4, wherein at least a portion of a valley of the support stent contacts at least a portion of a valley of the distal body stent in the contact region.

Clause 6. The branched stent graft of clause 4, wherein the support stent connects to the proximal body stent or the distal body stent in the contact region via a stent connector.

Clause 7. The branched stent graft of clause 6, wherein the stent connector is a weld or a crimp tube.

Clause 8. The branched stent graft of clause 1, wherein the support stent includes a curved portion having a profile conforming with a profile of the coupling.

Clause 9. A branched stent graft comprising:
a main body aligning with a blood vessel in a radially expanded state and extending between proximal and distal ends; and
a coupling extending from the main body proximate a branching blood vessel branching from the blood vessel in the radially expanded state;
the main body including a proximal body stent at least partially located between the proximal end of the main body and the coupling and a distal body stent at least partially located distal the coupling, the proximal body stent having a proximal body stent diameter, the distal body stent having a distal body stent diameter, the main body including a surrounding region located between the proximal and distal body stents and extending along a main body circumference, the main body further including a support stent located in the surrounding region and configured to maintain the main body in an open state when aligned with the blood vessel, the support stent having a support stent diameter, and the support stent diameter being less than the proximal body stent diameter and/or the distal body stent diameter.

Clause 10. The branched stent graft of any preceding clause, and in particular of clause 9, wherein the support stent diameter is in a range of 0.2 to 0.45 millimeters and the proximal body stent diameter and/or the distal body stent diameter are in a range of 0.50 to 0.8 millimeters.

Clause 11. The branched stent graft of any preceding clause, and in particular of clause 9, wherein the support stent is a wire form support stent.

Clause 12. The branched stent graft of any preceding clause, and in particular of clause 9, wherein the support stent is a laser cut support stent.

Clause 13. A branched stent graft comprising:
a main body aligning with a blood vessel in a radially expanded state and extending between proximal and distal ends; and
a coupling extending from the main body proximate a branching blood vessel branching from the blood vessel in the radially expanded state;
the main body including a bare body stent portion secured to the proximal end of the main body and a distal body stent portion at least partially located distal the coupling, the main body including a surrounding region located between the bare stent portion and the distal body stent portion and extending along a main body circumference, and the main body further including a loop stent portion located in the surrounding region and extending less than the main body circumference, and the loop stent portion configured to maintain the main body in an open state when aligned with the blood vessel.

Clause 14. The branched stent graft of any preceding clause, and in particular of clause 13, wherein the loop stent portion includes a closed perimeter having first and second ends.

Clause 15. The branched stent graft of any preceding clause, and in particular of clause 14, wherein the main body includes a cuff region surrounding the coupling and the first and second ends of the closed perimeter of the loop stent portion are spaced apart from the cuff region along the main body to provide flexibility to the coupling.

Clause 16. The branched stent graft of any preceding clause, and in particular of clause 13, wherein the loop stent portion is not directly connected to the bare body stent portion or the distal body stent portion.

Clause 17. The branched stent graft of any preceding clause, and in particular of clause 13, wherein a contacting loop stent portion of the loop stent portion contacts a contacting stent portion of the bare body stent portion or the distal body stent portion in a contact region.

Clause 18. The branched stent graft of any preceding clause, and in particular of clause 13, wherein the bare body stent portion, the distal body stent portion, and the loop stent portion form a continuous stent.

Clause 19. The branched stent graft of any preceding clause, and in particular of clause 18, wherein the bare body stent portion and the loop stent portion are connected with a proximal bridge portion and the loop stent portion and the distal body stent portion are connected through a distal bridge portion.

Clause 20. The branched stent graft of any preceding clause, and in particular of clause 13, wherein the bare body stent portion having a bare body stent portion diameter, the distal body stent portion having a distal body stent portion diameter, the loop stent portion having a loop stent portion diameter, and the loop stent portion diameter being less than the bare body stent diameter and/or the distal body stent diameter.

## Claims

1. A branched stent graft comprising:
a main body aligning with a blood vessel in a radially expanded state and extending between proximal and distal ends; and
a coupling extending from the main body proximate a branching blood vessel branching from the blood vessel in the radially expanded state;
the main body including a proximal body stent at least partially located between the proximal end of the main body and the coupling and a distal body stent at least partially located distal the coupling, the main body including a surrounding region located between the proximal and distal body stents and extending along a main body circumference, the main body further including a support stent located in the surrounding region and configured to maintain the main body in an open state when aligned with the blood vessel, and the support stent imparts less radial force than the proximal and distal body stents.

2. The branched stent graft of claim 1, wherein the support stent extends along an entirety of the main body circumference.

3. The branched stent graft of any preceding claim, wherein the support stent extends partially between the coupling and the proximal body stent or the distal body stent.

4. The branched stent graft of any preceding claim, wherein a contacting support stent portion of the support stent contacts a contacting body stent portion of the proximal body stent or the distal body stent in a contact region.

5. The branched stent graft of claim 4, wherein at least a portion of a valley of the support stent contacts at least a portion of a valley of the distal body stent in the contact region.

6. The branched stent graft of claim 4, wherein the support stent connects to the proximal body stent or the distal body stent in the contact region via a stent connector; and optionally wherein the stent connector is a weld or a crimp tube.

7. The branched stent graft of any preceding claim, wherein the support stent includes a curved portion having a profile conforming with a profile of the coupling.

8. A branched stent graft comprising:
a main body aligning with a blood vessel in a radially expanded state and extending between proximal and distal ends; and
a coupling extending from the main body proximate a branching blood vessel branching from the blood vessel in the radially expanded state;
the main body including a proximal body stent at least partially located between the proximal end of the main body and the coupling and a distal body stent at least partially located distal the coupling, the proximal body stent having a proximal body stent diameter, the distal body stent having a distal body stent diameter, the main body including a surrounding region located between the proximal and distal body stents and extending along a main body circumference, the main body further including a support stent located in the surrounding region and configured to maintain the main body in an open state when aligned with the blood vessel, the support stent having a support stent diameter, and the support stent diameter being less than the proximal body stent diameter and/or the distal body stent diameter.

9. The branched stent graft of claim 8, wherein the support stent diameter is in a range of 0.2 to 0.45 millimeters and the proximal body stent diameter and/or the distal body stent diameter are in a range of 0.50 to 0.8 millimeters.

10. The branched stent graft of claim 8 or 9, wherein the support stent is a wire form support stent; and/or wherein the support stent is a laser cut support stent.

11. A branched stent graft comprising:
a main body aligning with a blood vessel in a radially expanded state and extending between proximal and distal ends; and
a coupling extending from the main body proximate a branching blood vessel branching from the blood vessel in the radially expanded state;
the main body including a bare body stent portion secured to the proximal end of the main body and a distal body stent portion at least partially located distal the coupling, the main body including a surrounding region located between the bare stent portion and the distal body stent portion and extending along a main body circumference, and the main body further including a loop stent portion located in the surrounding region and extending less than the main body circumference, and the loop stent portion configured to maintain the main body in an open state when aligned with the blood vessel.

12. The branched stent graft of claim 11, wherein the loop stent portion includes a closed perimeter having first and second ends; and optionally wherein the main body includes a cuff region surrounding the coupling and the first and second ends of the closed perimeter of the loop stent portion are spaced apart from the cuff region along the main body to provide flexibility to the coupling.

13. The branched stent graft of claim 11 or 12, wherein the loop stent portion is not directly connected to the bare body stent portion or the distal body stent portion; and/or wherein a contacting loop stent portion of the loop stent portion contacts a contacting stent portion of the bare body stent portion or the distal body stent portion in a contact region.

14. The branched stent graft of any one of claims 11 to 13, wherein the bare body stent portion, the distal body stent portion, and the loop stent portion form a continuous stent; and/or wherein the bare body stent portion and the loop stent portion are connected with a proximal bridge portion and the loop stent portion and the distal body stent portion are connected through a distal bridge portion.

15. The branched stent graft of any one of claims 11 to 14, wherein the bare body stent portion having a bare body stent portion diameter, the distal body stent portion having a distal body stent portion diameter, the loop stent portion having a loop stent portion diameter, and the loop stent portion diameter being less than the bare body stent diameter and/or the distal body stent diameter.
